# EUROPEAN PATENT SPECIFICATION

(11) **EP 3 858 262 B1**
(45) Date of publication and mention of the grant of the patent: **16.11.2022**
(21) Application number: 19866573.9
(22) Date of filing: 20.09.2019
(51) Int. Cl.: A61B 17/12, A61F 2/97, A61B 17/00, A61F 2/95, A61B 90/00

(54) **ELECTRICAL DETACHMENT MECHANISM AND ELECTRICAL DETACHMENT DEVICE**
MECHANISMUS ZUR ELEKTRISCHEN TRENNUNG UND VORRICHTUNG ZUR ELEKTRISCHEN TRENNUNG
MÉCANISME DE RETRAIT ÉLECTRIQUE ET DISPOSITIF DE RETRAIT ÉLECTRIQUE

(30) Priority: 30.09.2018 CN 201811170257
(43) Date of publication of application: 04.08.2021
(73) Proprietor: MicroPort NeuroTech (Shanghai) Co., Ltd., Shanghai 201318 (CN)
(72) Inventor: SONG, Li, Shanghai 201318 (CN); GUO, Yuanyi, Shanghai 201318 (CN); CHEN, Bing, Shanghai 201318 (CN); CHANG, Mengqi, Shanghai 201318 (CN); WANG, Yiqun Bruce, Shanghai 201318 (CN)
(74) Representative: Patentship Patentanwaltsgesellschaft mbH
(86) International application number: PCT/CN2019/106824
(87) International publication number: WO 2020/063455

(56) References cited:
- WO-A1-2012/166804
- US-A1- 2005 090 861
- US-A1- 2015 066 073

## Description

### TECHNICAL FIELD

The present invention relates to the field of medical instrument and, in particular, to an electrolytic detachment mechanism and an electrolytic detachment device.

### BACKGROUND

An intracranial vascular malformation is a tumor-like bulge in the wall of a blood vessel caused by an abnormal change in the blood vessel. In particular, for patients with intracranial aneurysms, when a sudden rise in blood pressure occurs, the aneurysm may rupture, resulting in disabling or fatal hemorrhage. The treatment of an intracranial aneurysm with a Guglielmi detachable coil (electrolytically detachable coil) was first reported in 1991. Since then, with the development of materials and therapeutic equipment, coil-based embolization has become the first choice therapy for intracranial aneurysms.

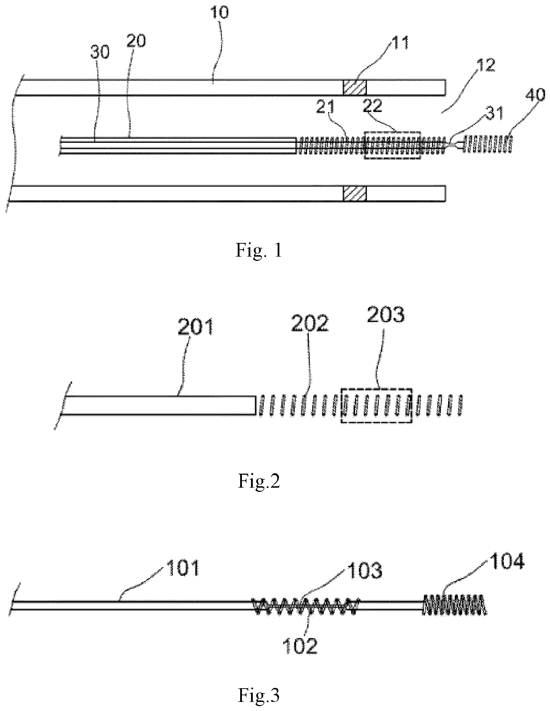

Reference is now to Fig. 1, a schematic cross-sectional view of an existing electrolytically detachable coil, which includes a microcatheter 10, a pusher rod 20, a conductive wire 30, a coil 40 and a detachable joint 31.A distal opening 12 of the microcatheter 10 is configured to be disposed close to the neck of an aneurysm. The pusher rod 20 is inserted within the microcatheter 10, and the conductive wire 30 is in turn disposed within the pusher rod 20. A proximal end of the conductive wire 30 is electrically connected to an external detachment apparatus (not shown), and a distal end thereof is connected to the coil 40 via the detachable joint 31. A distal end portion of the pusher rod 20 is provided with an elastic member 21 configured to make the distal end portion softer and easier to pass through curved intracranial blood vessels. The microcatheter 10 has a first radiopaque section 11 at a distal end thereof, and the elastic member 21 has a second radiopaque section 22. A physician can determine where the pusher rod 20 is currently positioned in the microcatheter 10 during its advancement therein and thus determine whether the coil 40 has entered the lumen of the aneurysm, based on an observed positional relationship between the first and second radiopaque sections 11, 22.

As shown in Fig. 1, the coil 40 is detached usually when the first and second radiopaque sections 11, 22 together define a shape like the inverted letter T. That is, only when the second radiopaque section 22 moves toward the distal end of the microcatheter 10 and completely passes through the first radiopaque section 11, can it be ensured that the detachable joint 31 extends out of the microcatheter 10 from the distal opening 12 and comes into contact with the blood, an environment allowing electrolytic detachment of the detachable joint 31. Only then can the detachable joint 31 be energized to detach the coil 40. However, the inventors have found that, due to tolerances of the microcatheter 10 and the pusher rod 20, even when an inverted T-shaped configuration formed by the first and second radiopaque sections 11, 22, there is still a chance that the detachable joint 31 has not been completely pushed out of the microcatheter 10 from the distal opening 12. In this case, the physician has to try several times before the coil 40 can be successfully detached. Moreover, as an environment for electrolytic detachment is unstable after contacting with the blood (forming a random electrolytic detachment environment because of blood- and thrombus-related factors), this may lead to a very long time required by the detachment. Further, it is also possible for the detachable joint 31 to extend too much out of the microcatheter 10 from the distal opening 12. In this case, it is very likely for the detachable joint 31 and the portion of the pusher rod 20 that has protruded out from the distal opening 12 to poke or even possibly rupture the aneurysm. Furthermore, when using the pusher rod 20 to advance the undetached coil 40 out of the distal opening 12 of the microcatheter 10, the entire detachment zone including a proximal end portion of the coil 40 may sometimes cause dislodgement of the microcatheter 10 from the lumen of the aneurysm. This so-called "recoil" effect is sometimes dangerous.

WO 2012/166804 A1 discloses a known electrolytic detachment mechanism with anodic and cathodic conductive elements.

Therefore, there is a need to develop a novel electrolytic detachment mechanism and electrolytic detachment device, which can overcome the problems of required multiple detachment attempts and the possibly dangerous extension of the detachable joint from the microcatheter required by the electrolytic detachment of existing electrolytically detachable coils.

### SUMMARY OF THE INVENTION

It is an object of the present invention to provide an electrolytic detachment mechanism and electrolytic detachment device, which can overcome the problems of low detachment reliability and a possibly dangerous, excessively long length of extension out of the microcatheter arising from the use of existing electrolytic detachment devices.

The above object is attained by an electrolytic detachment mechanism for cooperation with an electrolytic detachment apparatus to achieve an electrolytic detachment of an implant provided in the present invention, which comprises:
the implant;
a detachment member having a distal end coupled to the implant, wherein the detachment member is configured to be energized and electrolytically dissolved, thereby eliminating the coupling between the implant and the detachment member;
an electrical conduction member, comprising:
   an anodic conductive element covered by a first insulating element, wherein the anodic conductive element has a distal end coupled to a proximal end of the detachment member and a proximal end configured to be coupled to a positive terminal of the electrolytic detachment apparatus; and
   a cathodic conductive element having a proximal end configured to be coupled to a negative terminal of the electrolytic detachment apparatus, the cathodic conductive element electrically insulated from the anodic conductive element by the first insulating element; and
   an absorption member configured to, when absorbing electrolytes, provide an electrical conduction between the detachment member and the cathodic conductive element.

Optionally, the absorption member may be made of a hydrogel material.

Optionally, the absorption member may surround the detachment member.

Optionally, the absorption member may be a spiral structure or a hollow tube sleeved over the detachment member.

Optionally, the absorption member may be a coating over the detachment member.

Optionally, the hydrogel material may be one or a combination of more than one selected from: hydrogel based on cellulose and derivatives thereof; gelatin-modified hydrogels; cross-linked hydrogels based on chitosan and derivatives thereof; cross-linked hydrogels based on hyaluromic acid and modified forms thereof; cross-linked hydrogels based on polyethylene glycol and derivatives thereof; cross-linked hydrogels based on poly(vinyl alcohol) and derivatives thereof; cross-linked hydrogels based on poly(N-methylpyrrolidone) and derivatives thereof; polyester-based hydrogels; cross-linked hydrogel based on polyacrylamide and derivatives thereof; cross-linked swellable polymers derived from one or more polymerizable unsaturated carboxylic acid monomers containing olefinic bonds; and hydrogel based on hydroxyethyl methacrylate and derivatives thereof.

The above object is attained by an electrolytic detachment device provided in the present invention, which comprises the electrolytic detachment mechanism as defined above. The electrolytic detachment device further comprises:
a catheter; and
a pusher rod coupled to a proximal end of the electrolytic detachment mechanism,
wherein each of the electrolytic detachment mechanism and the pusher rod is moveably received in the catheter, and wherein the pusher rod is configured to cooperate with the catheter to deliver the electrolytic detachment mechanism to a target site.

Optionally, the pusher rod may be provided at a distal end thereof with a flexible member, and wherein the pusher rod is coupled to the electrolytic detachment mechanism by the flexible member.

Optionally, each of the pusher rod and the flexible member may be hollow a structure, wherein the electrical conduction member is received in the pusher rod and in the flexible member, wherein the cathodic conductive element is covered by a second insulating element to insulate the pusher rod from the flexible member, and wherein the cathodic conductive element has a distal end exposed from the second insulating element and electrically connectable to the detachment member by the absorption member.

Optionally, each of the pusher rod and the flexible member may be a hollow structure, wherein the anodic conductive element is received in the pusher rod and in the flexible member, with the pusher rod and the flexible member together configured as the cathodic conductive element.

Optionally, the absorption member and the flexible member may be arranged side by side along an axial direction, and wherein the absorption member is located on a distal end of the flexible member.

Optionally, a proximal end portion of the absorption member may be received in the flexible member, with a distal end of the absorption member protruding out of the flexible member from a distal end of the flexible member.

Optionally, the absorption member may be entirely received in the flexible member.

Optionally, the flexible member may have a first radiopaque section, with the catheter having a second radiopaque section at a distal end thereof, wherein each of the first and second radiopaque sections is made of a radiopaque material.

The provided electrolytic detachment mechanism and electrolytic detachment device offer the following benefits:
first, when absorbing electrolytes, the absorption member in the electrolytic detachment mechanism will maintain electrical conduction between the detachment member and the cathodic conductive element, thereby creating a stable electrolytic detachment environment allowing electrolytic dissolution of the detachment member. With this design, when the electrical conduction member is energized, the detachment member coupled to the anodic conductive element will react electrochemically with the cathodic conductive element and be thus electrolytically dissolved, resulting in detachment of the implant from the detachment member and hence from the whole electrolytic detachment mechanism. Compared with the prior art, since the absorption member can maintain electrical conduction between the detachment member and the cathodic conductive element and provide a stable electrolysis environment when absorbing electrolytes, enhanced electrolytic detachment reliability can be attained, the problems of a long detachment time and required multiple detachment attempts with existing electrolytic detachment devices can be overcome, and increased reliability of safe detachment can be achieved.
second, since the electrolytic detachment device incorporates the electrolytic detachment mechanism, it allows electrolytic detachment of the detachment member within the catheter, dispensing with the need to push the detachment member out of the catheter from the opening at the distal thereof, that is, it is ensured that the detachment member can come into contact with electrolytes to form a stable microcirculatory electrolytic detachment environment, in this way, electrolytic detachment of the implant can be caused anywhere within the catheter in a safe and effective manner, thus preventing the problems of a possibly dangerous, excessively long length of extension of the implant out of the catheter from the distal opening thereof and the occurrence of a "recoil" effect and resulting in significantly increased safety during implantation of the electrolytic detachment device.
third, in practical operation of the electrolytic detachment device, physiological saline or the like which contains electrolytes is dropwise added into the catheter, ensuring that there are always electrolytes available to the absorption member and thus maintaining a stable microcirculatory environment allowing electrolytic detachment of the detachment member. This overcomes the problems of a long detachment time and required multiple detachment attempts with existing electrolytic detachment devices and results in increased reliability of safe detachment. Moreover, since the electrolytic detachment of the implant can be caused when the pusher rod is being advanced toward the distal end, without needing to confirm the formation of an "inverted T-like" shape in a fluoroscopic image, operation of the physician can be made easier. Alternatively, the electrolytic detachment may be caused after the pusher rod has been pushed to a desired position. These arrangements may be combined in various ways to address different surgical conditions and complex surgical environments.

### BRIEF DESCRIPTION OF THE DRAWINGS

It will be appreciated by those of ordinary skill in the art that the accompanying drawings are provided for a better understanding of the present invention and do not limit it in any way. In these figures:
Fig. 1 is a schematic cross-sectional view of an existing electrolytically detachable coil;
Fig. 2 is a schematic illustration of a pusher rod provided at a distal end thereof with a flexible member according to an embodiment of the present invention;
Fig. 3 is a schematic illustration of an electrolytic detachment mechanism according to an embodiment of the present invention, which includes an absorption member fabricated of a hydrogel material into a spring;
Fig. 4 is a schematic cross-sectional view of an electrolytic detachment device according to an embodiment of the present invention, which includes an absorption member entirely disposed on a distal side of a flexible member and not overlapped with the flexible member;
Fig. 5 is a schematic cross-sectional view of an electrolytic detachment device according to an embodiment of the present invention, which includes an absorption member having a proximal end portion received within a flexible member and a distal end protruding out of the flexible member;
Fig. 6 is a schematic cross-sectional view of an electrolytic detachment device according to an embodiment of the present invention, which includes an absorption member entirely inserted in a flexible member;
Fig. 7 is a schematic illustration of an electrolytic detachment mechanism according to an embodiment of the present invention, which includes an absorption member fabricated of a hydrogel material into a coating;
Fig. 8 is a schematic illustration of an electrolytic detachment mechanism according to an embodiment of the present invention, which includes an absorption member fabricated of a hydrogel material into a tube; and
Fig. 9 is a schematic cross-sectional view of the tube of Fig. 8 taken along line a-a.

In these figures,
10: a microcatheter; 11: a first radiopaque section; 12: a distal opening; 20: a pusher rod; 21: an elastic member; 22: a second radiopaque section; 30: a conductive wire; 31: a detachable joint; 40: a coil;
101: an electrical conduction member; 102: an absorption member; 102': a coating; 102": a tube; 103: a detachment member; 104: a coil; 201: a pusher rod; 202: a flexible member; 203: a first radiopaque section; 301: a catheter; 302: a second radiopaque section; and 303: a distal end of the catheter.

### DETAILED DESCRIPTION

The above and other objects, advantages and features of the present invention will become more apparent from the following detailed description of several specific embodiments thereof, which is to be read in conjunction with the accompanying drawings. It is noted that the figures are provided in a very simplified form not necessarily presented to scale, with their only intention to facilitate convenience and clarity in explaining the disclosed embodiments. In addition, structures shown in the figures are usually a part of actual structures. In particular, as the figures tend to have distinct emphases, they are often drawn to different scales.

As used herein and in the appended claims, the singular forms "a", "an", and "the" include plural references unless the context clearly dictates otherwise. As used herein and in the appended claims, the term "or" is generally employed in its sense including "and/or" unless the content clearly dictates otherwise. The term "proximal" generally refers to the end of an object closer to a physician, and "distal" generally refers to the end closer to a lesion of a patient.

The core concept of the present invention is to provide an electrolytic detachment mechanism for cooperating with an electrolytic detachment apparatus to allow the electrolytic detachment of an implant, which includes the implant, a detachment member, an electrical conduction member and an absorption member. Compared with the prior art, the absorption member is configured to absorb electrolytes and thereby remain electrically conductive with a cathodic conductive element. This can ensure reliable electrolytic detachment, thereby overcoming the problems of a long detachment time and required multiple detachment attempts with existing electrolytic detachment devices.

The present invention also provides an electrolytic detachment device comprising the electrolytic detachment mechanism, a catheter and a pusher rod. In practical operation, the detachment member can be electrolytically detached within the catheter, dispensing with the need to push the detachment member out of the catheter from a distal opening of the catheter. It can be ensured that the detachment member is brought into contact with electrolytes to create a microcirculatory electrolytic detachment environment allowing the implant to be electrolytically detached anywhere in the catheter in a safe and effective manner. This can result in increased safety and reliability of the electrolytic detachment device during implantation by preventing a possibly dangerous, excessively long length of extension of the implant out of the catheter and avoiding the occurrence of a "recoil" effect.

More specifically, during the implantation of the implant into the body of a patient, through drop-wise filling the catheter with physiological saline or another electrolyte solution suited to be added to the patient's body, allowing the absorption member always absorbing the electrolytes, ensuring the detachment member always have a microcirculatory environment for electrolytic detachment . This prevents the problems of a long detachment time and required multiple detachment attempts associated with existing electrolytic detachment devices, resulting increased detachment reliability. Moreover, since the electrolytic detachment can be done anytime during advancement of the pusher rod toward the distal end of the catheter without needing to confirm an "inverted T-like" shape in the fluoroscopic image, the physician's operation can be made easier.

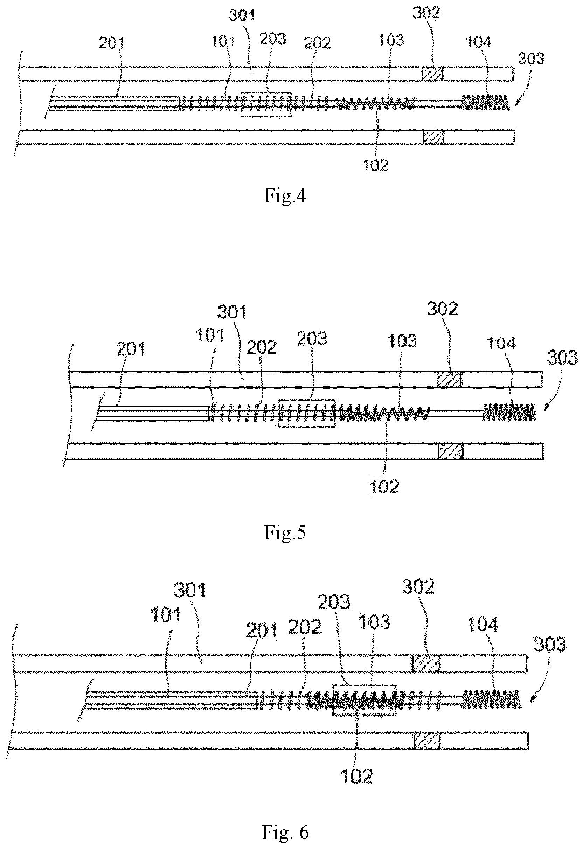

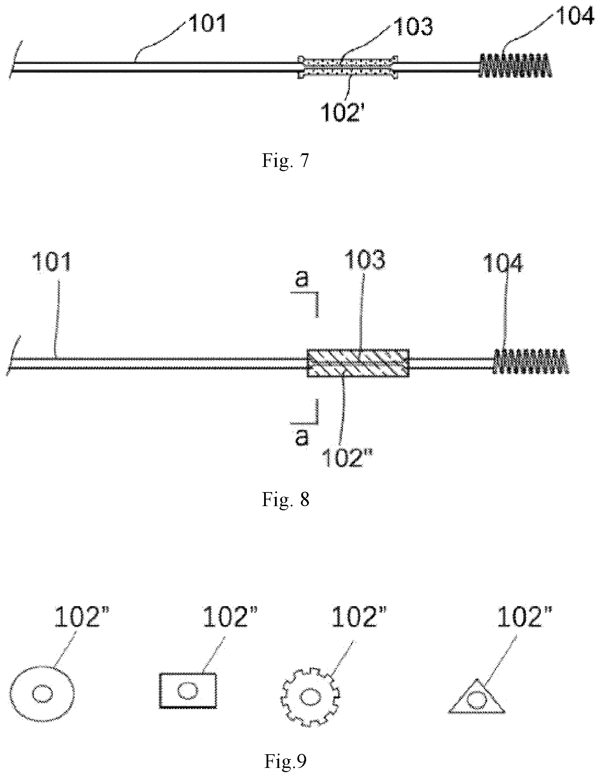

A detailed description is set forth below with reference to the accompanying drawings. Fig. 2 is a schematic illustration of a pusher rod provided at a distal end thereof with a flexible member according to an embodiment of the present invention. Fig. 3 is a schematic illustration of an electrolytic detachment mechanism according to an embodiment of the present invention. Figs. 4 to 6 are schematic cross-sectional views of electrolytic detachment devices according to preferred embodiments of the present invention. Figs. 7 and 8 are schematic illustrations of electrolytic detachment mechanisms according to preferred embodiments of the present invention. Fig. 9 is a schematic cross-sectional view of a tube of Fig. 8 taken along line a-a.

First of all, referring to Fig. 3, the illustrated electrolytic detachment mechanism can be used for embolization of a vascular malformation, in particular, an intracranial aneurysm, and includes an implant, a detachment member 103, an electrical conduction member 101 and an absorption member 102. The implant is configured to be deployed at a target site. In particular, the implant may be a coil 104 configured for embolization of a vascular malformation. The detachment member 103 is coupled at one end to the coil 104, and when the detachment member 103 is electrolytically dissolved, the coupling between the coil 104 and the detachment member 103 will be eliminated. Preferably, the detachment member 103 may be made of a biocompatible active metal such as Mg (magnesium), Zn (zinc), Fe (iron) or the like. Of course, the detachment member 103 may be optionally made of a biocompatible active alloy such a magnesium zinc alloy, a magnesium iron alloy, stainless steel, etc.

The electrical conduction member 101 includes an anodic conductive element and a cathodic conductive element, which are separated from each other, and the anodic conductive element is covered by a first insulating element. A distal end of the anodic conductive element is coupled to the other end of the detachment member 103, and a proximal end of the anodic conductive element is coupled to a positive terminal of an external electrolytic detachment apparatus. A proximal end of the cathodic conductive element is coupled to a negative terminal of the external electrolytic detachment apparatus. The external electrolytic detachment apparatus with the positive and negative terminals is configured to provide an electrical current required for electrolysis to take place in the electrolytic detachment mechanism. It may have a conventional structure, a detailed description thereof is deemed unnecessary. The first insulating element is configured to electrically insulate the anodic conductive element from the cathodic conductive element.

In particular, the absorption member 102 is configured to, when absorbing electrolytes, provide electrical conduction between the detachment member 103 and the cathodic conductive element, thus enabling electrolytic dissolution of the detachment member 103. The absorption member 102 may not be directly coupled to the cathodic conductive element, the detachment member 103 or the anodic conductive element. Rather, it may be configured to swell or chemically absorb electrolytes so that an electrolyte solution provides electrical conduction between the detachment member 103 and the cathodic conductive element. Alternatively, for ease of configuration, the absorption member 102 may be coupled to one of the cathodic conductive element, the detachment member 103 and the anodic conductive element, or to the coil 104. Additionally, it may also be coupled to the pusher rod 201 or the flexible member 202, as detailed below, so as to be located at a relatively fixed position within the whole electrolytic detachment device, where it can absorb electrolytes and perform the desired function.

Specifically, the anodic conductive element may be implemented as a conductive wire and the first insulating element covering it as an insulating layer that electrically insulates the conductive wire from the cathodic conductive element. The detachment member 103 may be implemented as an electrical conductor that is coupled to the anodic conductive element and exposed to the external environment. In this way, when the absorption member 102 absorbs electrolytes, electrical conduction is provided between the detachment member 103 and the cathodic conductive element. It is to be noted that, according to the present invention, the implant is not limited to the coil 104, as it may also be a stent, a prosthetic valve, an occluder or another implantable instrument for interventional treatment.

Thus, when the absorption member 102 absorbs electrolytes, it establishes electrical conduction between the detachment member 103 and the cathodic conductive element and creates an electrolytic detachment environment where the detachment member 103 can be electrolytically dissolved. It should be understood that the electrical conduction is not provided by a direct contact or connection between the detachment member 103 and the cathodic conductive element but by electrolytes absorbed by the absorption member 102. That is, the electrical conduction between the detachment member 103 and the cathodic conductive element is made by electrolytes. The electrolytes may be contained, for example, in the blood, physiological saline or another biocompatible electrolyte solution, and the absorption member 102 will become electrically conductive when absorbing such electrolytes, thus establishing electrical conduction between the detachment member 103 and the cathodic conductive element. Since the detachment member 103 is coupled to the anodic conductive element, when the electrical conduction member 101 is energized, the detachment member 103 will electrochemically react with the cathodic conductive element. As a result, the detachment member 103 as the anode will be electrolytically dissolved, eliminating the coupling between the coil 104 and the detachment member 103. The disconnected coil 104 will leave the electrolytic detachment mechanism and facilitate thrombus formation in the lumen of the aneurysm. Compared with the prior art, as the absorption member 102 can maintain electrical conduction between the detachment member 103 and the cathodic conductive element when it has absorbed electrolytes, more reliable electrolytic detachment can be achieved and the problem of a long detachment time and required multiple detachment attempts for the existing electrolytically detachable coils due to an instable electrolysis environment can be overcome. Moreover, improved reliability of safe detachment can be achieved and the problem of low detachment reliability with existing electrolytically detachable coils can be solved.

The absorption member 102 may swell or absorb electrolytes so that an electrolyte solution electrically connects the detachment member to the cathodic/anodic conductive element. For example, it may be formed of a hydrogel material or another material with chemical adsorption properties. Here, the hydrogel material refers to a polymer, which swells when absorbing water and has very good water retention properties. Specifically, the hydrogel material may include hydrogels based on natural polymers and synthetic organic polymers. The hydrogel material may in particular include, but is not limited to, one or a combination of more selected from: hydrogel based on cellulose and derivatives thereof; gelatin-modified hydrogels; cross-linked hydrogels based on chitosan and derivatives thereof; cross-linked hydrogels based on hyaluromic acid and modified forms thereof; cross-linked hydrogels based on polyethylene glycol and derivatives thereof; cross-linked hydrogels based on poly(vinyl alcohol) and derivatives thereof; cross-linked hydrogels based on poly(N-methylpyrrolidone) and derivatives thereof; polyester-based hydrogels; cross-linked hydrogel based on polyacrylamide and derivatives thereof; hydrogel based on hydroxyethyl methacrylate and derivatives thereof; cross-linked swellable polymers derived from one or more polymerizable unsaturated carboxylic acid monomers containing olefinic bonds; and so on.

Further, the absorption member 102 may be made of the hydrogel material into one of many possible shapes. For example, it may be fabricated as a spiral structure such as a spring (as shown in Fig. 3), which is wound over the detachment member 103 and configured to be electrically connected to the cathodic conductive element when absorbing electrolytes. Specifically, a hydrogel fiber or filament may be coiled into a spiral micro-spring over the detachment member 103. That is, the spring may be entirely disposed over the detachment member 103. Preferably, the micro-spring over the detachment member 103 may have a section designed to have sparse turns which, on the one hand, allow exposure of the detachment member 103 to electrolytes and, on the other hand, provide a margin for accommodating expansion of the micro-spring. In this way, when the hydrogel fiber or filament absorbs electrolytes, it will swell and come into contact with both the detachment member 103 and the cathodic conductive element, thus establishing electrical conduction between the detachment member 103 and the cathodic conductive element.

In other embodiments, the absorption member 102 may be fabricated of the hydrogel material into a coating 102' (as shown in Fig. 7) over the detachment member 103. Specifically, the hydrogel material may be coated on a surface (preferably, an outer surface) of the detachment member 103. Here, the coating may include partial or complete coating of the surface of the detachment member 103. The coating 102' made of the hydrogel material can also swell and come into contact with the cathodic conductive element when absorbing electrolytes, thereby making electrical conduction between the detachment member 103 and the cathodic conductive element. In other embodiments, the absorption member 102 may be fabricated of the hydrogel material into a tube 102" (as shown in Fig. 8). The tube 102" is sleeved over and surrounds the detachment member 103. The tube 102" may have a cross section in the shape of a regular circle (as shown in Fig. 9(A)), a rectangle (as shown in Fig. 9(B)), a tooth gear (as shown in Fig. 9(C)) or a triangle (as shown in Fig. 9(D)). Alternatively, the shape of the cross section may also be otherwise polygonal or irregular. The tube 102" may swell and come into contact with the cathodic conductive element when absorbing electrolytes, thus establishing electrical conduction between the detachment member 103 and the cathodic conductive element.

Referring to Figs. 2 to 4, an electrolytic detachment device according to an embodiment of the present invention includes the electrolytic detachment mechanism as defined above. The electrolytic detachment device further includes a catheter 301 and a pusher rod 201. Both the electrolytic detachment mechanism and the pusher rod 201 are moveably received in the catheter 301, and the pusher rod 201 is configured to cooperate with the catheter 301 to deliver the electrolytic detachment mechanism to a target site in the patient's body, such as the lumen of the aneurysm. As the electrolytic detachment device according to this embodiment incorporates the above-described electrolytic detachment mechanism, the detachment member 103 can be electrolytically detached within the catheter 301 without needing to push the detachment member 103 out of the catheter from an opening at a distal end 303 of the catheter. It can be ensured that the detachment member 103 is brought into contact with electrolytes to create a microcirculatory electrolytic detachment environment allowing the coil 104 to be electrolytically detached anywhere in the catheter 301 in a safe and effective manner. This can prevent a possibly dangerous, excessively long length of extension of the coil 104 out of the opening at the distal end 303 of the catheter. Preferably, the coil 104 may be electrolytically detached within a portion of the catheter 301 around the distal end 303, thereby avoiding the "recoil" effect problem which is caused by: when the coil 104 extends out of the opening at distal end 303 of the catheter, the opening at the distal end 303 of catheter dislodges from the lumen of the aneurysm under the action of resisting forces applied by the wall of the lumen of the aneurysm to the detachment member such as the coil 104 occurs. As a result, increased safety can be achieved during the implantation of the electrolytic detachment device. In general, when the electrolytic detachment device is used to treat an intracranial aneurysm, it may be necessary to implant multiple such embolization coils 104. In this case, when a coil is electrolytically detached in the way as described above, the next coil to be subsequently electrolytically detached may push the already detached one out of the catheter from the opening at the distal end 303 and into the lumen of the aneurysm. This is because the coils 104 themselves are elastic, so when a first one of them that has been detached is located around the distal end 303 of the catheter without successfully entering the lumen of the aneurysm, a second coil can push it distally into the lumen of the aneurysm under the action of the pusher rod 201. In this way, enhanced prevention of a "recoil" effect can be achieved, resulting in increased safety during the implantation of the electrolytic detachment device.

Preferably, the pusher rod 201 may be provided at a distal end thereof with a flexible member 202 made of a flexible material or having a flexible structure. For example, it may be structured as a spring. Additionally, the flexible member 202 may be coupled to the electrolytic detachment mechanism so as to be able to push the electrolytic detachment mechanism. The flexible member 202 is provided to impart higher softness to a distal end portion of the pusher rod 201, which makes the portion easier to pass through curved intracranial blood vessels.

The structure of the electrolytic detachment device will be described in detail below with reference to Fig. 4. The pusher rod 201 and the flexible member 202 may be both hollow structures. For example, the pusher rod 201 may be implemented as a stainless steel hollow tube and the flexible member 202 as a stainless steel spring. Preferably, the electrical conduction member 101 of the electrolytic detachment mechanism is made up of two conductive wires. That is, the anodic and cathodic conductive elements are both conductive wires. These conductive wires may be both inserted in the pusher rod 201 and each coated with an insulating layer. That is, in addition to the first insulating element coating the anodic conductive element in the electrolytic detachment mechanism, the cathodic conductive element may be coated with a second insulating element for electrically insulating the anodic and cathodic conductive elements from each other. The cathodic conductive element may have a distal end portion close to the detachment member 103, which is exposed from the second insulating element and can be brought into contact with the absorption member 102 that has absorbed electrolytes. With this arrangement, the absorption member 102 can establish electrical conduction between the cathodic conductive element and the detachment member 103. Preferably, the electrical conduction member 101 is covered by a third insulating element. That is, in addition to the first insulating element covering the anodic conductive element and the second insulating element covering the cathodic conductive element, both of them are additionally covered by the third insulating layer, which further ensures electrical insulation of the electrical conduction member 101 from the pusher rod 201. In alternative embodiments, only the anodic conductive element of the electrolytic detachment mechanism may be inserted in the pusher rod 201 and the flexible member 202, with the pusher rod 201 and the flexible member 202 together configured as the cathodic conductive element. In this case, the flexible member 202 may have a distal end close to the detachment member 103, which can be brought into contact with the absorption member 102 that has absorbed electrolytes. With this arrangement, the absorption member 102 can also establish electrical conduction between the cathodic conductive element made up of the pusher rod 201 and the flexible member 202 and the detachment member 103. The first insulating element covering the anodic conductive element can ensure electrical insulation of the pusher rod 201 from the flexible member 202. When the external electrolytic detachment apparatus provides an electrical current to the electrical conduction member 101, the electrical current will flow from the positive terminal of the electrolytic detachment apparatus to the detachment member 103 via the anodic conductive element, then to the flexible member 202 via the absorption member 102 that has absorbed electrolytes, then to the pusher rod 201 via the flexible member 202 and finally to the negative terminal of the electrolytic detachment apparatus via the pusher rod 201, thus, an electrical current loop is formed. Under the action of this electrical current, the detachment member 103 will be electrochemically corroded and break, resulting in detachment of the coil 104.

Preferably, the absorption member 102 and the flexible member 202 may be arranged side by side along an axial direction of the flexible member 202, and the absorption member 102 may be arranged closer to a distal end of the flexible member 202. As such, there is no overlap between the absorption member 102 and the flexible member 202 along the axial direction of the flexible member 202. As shown in Fig. 4, the absorption member 102 may be disposed on the distal side of the flexible member 202 and offset from the flexible member 202 without any overlap therewith along the axial direction of the flexible member 202. This is suitable for the case of the electrical conduction member 101 being made up of the two conductive wires. Additionally, in this case, the detachment member 103 and the portion of the cathodic conductive element exposed from the second insulating element may be both surrounded by the absorption member 102. As such, when the absorption member 102 absorbs electrolytes, it will swell and come into contact with both the detachment member 103 and the portion of the cathodic conductive element exposed from the second insulating element. With this arrangement, the absorption member 102 is able to establish electrical conduction between the detachment member 103 and the cathodic conductive element.

In other embodiments, as shown in Fig. 5, the absorption member 102 may be partially inserted in the flexible member 202. Specifically, a proximal end portion of the absorption member 102 may be received in the flexible member 202, with a distal end of the absorption member 102 protruding out of the flexible member 202 from the distal end of the flexible member 202. As such, the absorption member 102 may be partially overlapped with the flexible member 202 along the axial direction of the flexible member 202.

In other embodiments, as shown in Fig. 6, the absorption member 102 may be entirely inserted in the flexible member 202 so that the absorption member 102 is completely overlapped with the flexible member 202 along the axial direction of the flexible member 202. It should be understood that, the term "completely overlapped" is meant to mean that the flexible member 202 may have a length greater than or equal to a length of the absorption member 102 and cover the total length of the absorption member 102 in the axial direction.

The arrangements with the absorption member 102 being partially or completely overlapped with the flexible member 202 along the axial direction of the flexible member 202 according to the above embodiments are suitable for the case of the cathodic conductive element being made up of the pusher rod 201 and the flexible member 202. In this case, the absorption member 102 houses only the detachment member 103 and is at least partially overlapped with the flexible member 202. Therefore, when the absorption member 102 absorbs electrolytes, it will swell and come into contact with both the detachment member 103 and the flexible member 202 that is configured as a component of the cathodic conductive element, thus establishing electrical conduction between them. Specifically, the absorption member 102 may be implemented as a micro-spring fabricated from a hydrogel fiber and the flexible member 202 as a stainless steel spring. The micro-spring may be partially or entirely arranged within the stainless steel spring, and the detachment member 103 may be in turn surrounded within the micro-spring.

In particular, in alternative embodiments, the pusher rod 201 may be implemented as a solid rod, which is made of, for example, stainless steel and is configured as the cathodic conductive element. In addition, the anodic conductive element of the electrical conduction member 101 may be implemented as a conductive wire covered with the first insulating element. In this case, rather than being inserted within the solid pusher rod, the electrical conduction member 101 may be arranged side by side radially with respect to the solid pusher rod. Preferably, the electrical conduction member 101 may be fastened at a number of points to the solid pusher rod, for example, by bonding, gluing or the like. In this case, the solid pusher rod may also be provided at a distal end thereof with the flexible member 202 that makes the distal end softer and facilitates its passage through tortuous blood vessels. In this case, the detachment member 103 may be arranged side by side radially with respect to the flexible member 202, and the absorption member 102 may be optionally made of a solid hydrogel material or a material with chemical adsorption properties. Opposing ends of the absorption member 102 may be brought into contact respectively with the detachment member 103 and the flexible member 202. With this arrangement, the absorption member 102 can also provide electrical conduction between the detachment member 103 and the cathodic conductive element. Of course, the detachment member 103 may also be inserted in the flexible member 202 as described above.

Referring to Fig. 4, in combination with Fig. 2, preferably, the flexible member 202 may have a first radiopaque section 203, and the catheter 301 may have a second radiopaque section 302 at the distal end 303 thereof. Both of the first and second radiopaque sections 203, 302 may be made of a radiopaque material so as to show positions thereof under X-Ray. For example, the first radiopaque section 203 may be made of a material different from the material of the rest of the flexible member 202 and of high radiopaqueness, such as platinum or another metal. In this way, under X-Ray fluoroscopy monitoring, the position and distance of the flexible member 202 relative to the distal end 303 of the catheter 301 can be monitored with a monitor. This can help the physician determine an appropriate timing for electrolytic detachment of the coil 104.

According to an embodiment, there is also provided a method for operating the electrolytic detachment device as defined above, which includes the steps of:
step i: pushing the catheter 301 to a target site in the patient's body (e.g., in the vicinity of a vascular malformation) and continuously drop-wise adding physiological saline into a proximal end of the catheter 301, which can be absorbed by the absorption member 102;
stepii: pushing the pusher rod 201 toward the distal end of the catheter 301 until a predetermined detachment location is reached;
stepiii: energizing the electrical conduction member 101 so that the detachment member 103 is electrolytically dissolved; and
stepiv: as a result of the electrolytic dissolution of the detachment member 103, detachment of the coil 104 from the detachment member 103, entry of the coil 104 into the lumen of the vascular malformation and thus embolization thereof.

Specifically, in step i, the physician may continuously adding physiological saline into the catheter 301 while manipulating the pusher rod 201. As a result, the absorption member 102 located over the detachment member 103 will absorb the physiological saline and swell, thus keeping the detachment member 103 in contacting with the cathodic conductive element and creating a conductive environment allowing electrolytic detachment of the detachment member 103. Compared with the prior art approach in which sufficient contact of the detachable joint with electrolytes in the blood or the like can be ensured to allow electrolytic detachment only after it has been pushed out of the microcatheter, in the method according to this embodiment, the absorption member 102 can be exposed to and continuously absorb electrolytes while it is being pushed and advanced. This can ensure a better conductive environment for electrolytic detachment, compared to the prior art, which can result in enhanced electrolytic detachment reliability and avoid the need for multiple electrolytic detachment attempts.

In step ii, the predetermined detachment location may depend on the patient's condition and is generally determined by the physician based on his/her own experience.

In step iii, the electrical conduction member 101 may be energized with an electrical current, so that the electrical current flows from the positive terminal of the external electrolytic detachment apparatus to the cathodic conductive element, further to the negative terminal of the electrolytic detachment apparatus through the anodic conductive element, the detachment member 103, the physiological saline, thereby forming an electrical current loop. Under the action of the electrical current, the detachment member 103 will be electrolytically dissolved, thereby detaching the coil 104.

Preferably, in step ii, as the pusher rod 201 is being pushed toward the distal end, the distance between the first and second radiopaque sections 203, 302 can be monitored by X-ray imaging. Upon the distance between the first and second radiopaque sections 203, 302 becoming not greater than a predetermined distance, pushing of the pusher rod 201 may be stopped. The predetermined distance may depend on the structure of the electrolytic detachment device and the requirements of the surgical procedure. The physician may determine the current position of the pusher rod 201by monitoring the position and distance of the first radiopaque section 203 with respect to the second radiopaque section 302 through X-ray imaging and compare it with the predetermined detachment location. Since sufficient contact of the detachment member 103 with electrolytes can be ensured to allow electrolytic detachment of the coil 104 without the need to pushing the detachment member 103 out of the catheter 301, it can be effectively ensured that the coil 104 can be electrolytically detached without a possibly dangerous, excessively long length of extension out of the catheter, and without a risk of the "recoil" effect. Thus, increased safety of the pushing operation can be achieved, and the problem of a required "inverted T-shaped" configuration conformed in the fluoroscopic image for the detachment of the coil associated with the prior art can be overcome.

Preferably, in step ii, the electrical conduction member 101 can be energized either when the pusher rod 201 is being pushed toward the distal end or when the distance between the first and second radiopaque sections 203, 302 is not greater than the predetermined distance and pushing of the pusher rod 201 has been stopped. The absorption member 102 will swell when coming into contact with the physiological saline drop-wise added to the catheter 301 or with the blood and maintain electrolytes (absorbed from the physiological saline or the blood). Therefore, it can be ensured that there is always a microcirculatory environment allowing electrolytic detachment of the detachment member 103, thus overcoming the problems of a long detachment time and required multiple detachment attempts associated with existing electrolytically detachable coils and achieving improved reliability of safe detachment. Preferably, pushing of the pusher rod 201 may be stopped when the electrolytic detachment mechanism gets close to the distal end 303 of the catheter, and the electrolytic detachment may be then caused. In this way, the coil 104 after electrolytical detachment can be around the opening of the catheter at the distal end 303. This can facilitate deployment of the coil 104 at a predetermined embolization site. Of course, in case of multiple coils 104 being required to be implanted, electrolytic detachment can be caused within the catheter 301, and a previously detached coil 104 can be pushed out by the next coil 104 to be subsequently detached, as required by the surgical procedure. Since the electrolytic detachment can be caused within the catheter 301 without needing to confirm the formation of an "inverted T-like" shape in a fluoroscopic image, this method can also enable easier operation of the physician by allowing him/her to start causing electrolytic detachment while the pusher rod 201 is being pushed toward the distal end. Of course, it is also possible for electrolytic detachment to be caused after the pusher rod 201 has been advanced to a desired position. These various arrangements may be combined in various ways to address different surgical conditions and complex surgical environments.

In summary, in the electrolytic detachment mechanism according to embodiments of the present invention, when absorbing electrolytes, the absorption member will maintain electrical conduction between the detachment member and the cathodic conductive element, thus creating a stable electrolytic detachment environment allowing electrolytic dissolution of the detachment member. With this design, when the electrical conduction member is energized, the detachment member coupled to the anodic conductive element with electrochemically react with the cathodic conductive element and will be thus electrolytically dissolved, resulting in detachment of the implant from the detachment member and hence from the whole electrolytic detachment mechanism and deployment thereof at the target site. Compared with the prior art, since the absorption member can maintain electrical conduction between the detachment member and the cathodic conductive element when absorbing electrolytes, enhanced electrolytic detachment reliability can be attained, the problems of a long detachment time and required multiple detachment attempts with existing electrolytic detachment devices can be overcome, and increased reliability of safe detachment can be achieved.

Further, since the electrolytic detachment device according to embodiments of the present invention incorporates the above-discussed electrolytic detachment mechanism, thus the electrolytic detachment of the detachment member is allowed within the catheter, dispensing with the need to push the detachment member out of opening at the distal of catheter, that is, ensuring the detachment member can come into contact with the electrolytes to form a stable microcirculatory electrolytic detachment environment, in this way, electrolytic detachment of the implant can be caused anywhere within the catheter in a safe and effective manner, thus preventing the problems of a possibly dangerous, excessively long length of extension of the implant out of the catheter from the distal opening thereof and the occurrence of a "recoil" effect and resulting in significantly increased safety during implantation of the electrolytic detachment device.

Furthermore, in practical operation of the electrolytic detachment device according to embodiments of the present invention, physiological saline is injected into the catheter, ensuring there are always electrolytes available to the absorption member, which maintain a stable microcirculatory environment allowing electrolytic detachment of the detachment member. This overcomes the problems of a long detachment time and required multiple detachment attempts with existing electrolytic detachment devices and results in increased reliability of safe detachment. Moreover, since the electrolytic detachment of the implant can be caused when the pusher rod is being advanced toward the distal end, without needing to confirm the formation of an "inverted T-like" shape in a fluoroscopic image, operation of the physician can be made easier. Multiple arrangements may be combined in various ways to address different surgical conditions and complex surgical environments.

The description presented above is merely that of some preferred embodiments of the present invention and does not limit the scope thereof in any sense. Any and all changes and modifications made by those of ordinary skill in the art based on the above teachings fall within the scope as defined in the appended claims.

## Claims

1. An electrolytic detachment mechanism for cooperation with an electrolytic detachment apparatus to achieve an electrolytic detachment of an implant, comprising:
the implant;
a detachment member (103) having a distal end coupled to the implant, wherein the detachment member (103) is configured to be energized and electrolytically dissolved, thereby eliminating the coupling between the implant and the detachment member (103);
an electrical conduction member (101), comprising:
an anodic conductive element covered by a first insulating element, wherein the anodic conductive element has a distal end coupled to a proximal end of the detachment member (103) and a proximal end configured to be coupled to a positive terminal of the electrolytic detachment apparatus; and
a cathodic conductive element having a proximal end configured to be coupled to a negative terminal of the electrolytic detachment apparatus, wherein the cathodic conductive element is electrically insulated from the anodic conductive element by
the first insulating element; **characterised in that** the electrolytic detachment mechanism further comprises
an absorption member (102) configured to, when absorbing electrolytes, provide an electrical conduction between the detachment member (103) and the cathodic conductive element.

2. The electrolytic detachment mechanism according to claim 1, wherein the absorption member (102) is made of a hydrogel material.

3. The electrolytic detachment mechanism according to claim 2, wherein the absorption member (102) surrounds the detachment member.

4. The electrolytic detachment mechanism according to claim 3, wherein the absorption member (102) is a spiral structure or a hollow tube sleeved over the detachment member (103).

5. The electrolytic detachment mechanism according to claim 2, wherein the absorption member (102) is coated over the detachment member.

6. The electrolytic detachment mechanism according to claim 2, wherein the hydrogel material is one or a combination of more than one selected from: hydrogel based on cellulose and derivatives thereof; gelatin-modified hydrogels; cross-linked hydrogels based on chitosan and derivatives thereof; cross-linked hydrogels based on hyaluromic acid and modified forms thereof; cross-linked hydrogels based on polyethylene glycol and derivatives thereof; cross-linked hydrogels based on poly(vinyl alcohol) and derivatives thereof; cross-linked hydrogels based on poly(N-methylpyrrolidone) and derivatives thereof; polyester-based hydrogels; cross-linked hydrogel based on polyacrylamide and derivatives thereof; cross-linked swellable polymers derived from one or more polymerizable unsaturated carboxylic acid monomers containing olefinic bonds; and hydrogel based on hydroxyethyl methacrylate and derivatives thereof.

7. An electrolytic detachment device comprising the electrolytic detachment mechanism according to any one of claims 1 to 6, wherein the electrolytic detachment device further comprises:
a catheter (301); and
a pusher rod (201) coupled to a proximal end of the electrolytic detachment mechanism,
wherein each of the electrolytic detachment mechanism and the pusher rod (201) is moveably received in the catheter (301), and wherein the pusher rod (201) is configured to cooperate with the catheter (301) to deliver the electrolytic detachment mechanism to a target site.

8. The electrolytic detachment device according to claim 7, wherein the pusher rod (201) is provided at a distal end thereof with a flexible member (202), and wherein the pusher rod (201) is coupled to the electrolytic detachment mechanism by the flexible member (202).

9. The electrolytic detachment device according to claim 8, wherein each of the pusher rod (201) and the flexible member (202) is a hollow structure, wherein the electrical conduction member (101) is received in the pusher rod (201) and in the flexible member (202), wherein the cathodic conductive element is covered by a second insulating element to insulate the pusher rod (201) from the flexible member (202), and wherein the cathodic conductive element has a distal end exposed from the second insulating element and electrically connectable to the detachment member (103) by the absorption member (102).

10. The electrolytic detachment device according to claim 8, wherein each of the pusher rod (201) and the flexible member (202) is a hollow structure, wherein the anodic conductive element is received in the pusher rod (201) and in the flexible member (202), with the pusher rod (201) and the flexible member (202) together configured as the cathodic conductive element.

11. The electrolytic detachment device according to claim 9 or 10, wherein the absorption member (102) and the flexible member (202) are arranged side by side along an axial direction, and wherein the absorption member (102) is located on a distal end of the flexible member (202).

12. The electrolytic detachment device according to claim 9 or 10, wherein the absorption member (102) has a proximal end portion received in the flexible member (202), and wherein the absorption member (102) has a distal end protruding out of a distal end of the flexible member (202).

13. The electrolytic detachment device according to claim 9 or 10, wherein the absorption member (102) is entirely received in the flexible member (202).

14. The electrolytic detachment device according to claim 8, wherein the flexible member (202) has a first radiopaque section (203) and a distal end of the catheter (303) has a second radiopaque section (302), and wherein each of the first and second radiopaque sections (203,302) is made of a radiopaque material.

## Patentansprüche

1. Elektrolytischer Trennungsmechanismus zum Zusammenwirken mit einer elektrolytischen Trennungsvorrichtung, um eine elektrolytische Trennung eines Implantats zu erreichen, umfassend:
das Implantat;
ein Trennungselement (103) mit einem an das Implantat gekoppelten distalen Ende, wobei das Trennungselement (103) ausgebildet ist, energetisiert und elektrolytisch aufgelöst zu werden, wodurch die Kopplung zwischen dem Implantat und dem Trennungselement (103) entfernt wird;
ein elektrisches Leitungselement (101), umfassend:
ein anodisch leitfähiges Element, das von einem ersten isolierenden Element bedeckt ist, wobei das anodisch leitfähige Element ein distales Ende aufweist, das mit einem proximalen Ende des Trennungselements (103) gekoppelt ist, und ein proximales Ende aufweist, das ausgebildet ist, mit einem positiven Anschluss der elektrolytischen Trennungsvorrichtung gekoppelt zu werden; und
ein kathodisch leitfähiges Element mit einem proximalen Ende, das ausgebildet ist, mit einem negativen Anschluss der elektrolytischen Trennungsvorrichtung gekoppelt zu werden, wobei das kathodisch leitfähige Element von dem anodisch leitfähigen Element durch das erste isolierende Element elektrisch isoliert ist;
**dadurch gekennzeichnet, dass** der elektrolytische Trennungsmechanismus ferner umfasst:
ein Absorptionselement (102), das ausgebildet ist, beim Absorbieren von Elektrolyten eine elektrische Leitung zwischen dem Trennungselement (103) und dem kathodisch leitfähigen Element bereitzustellen.

2. Elektrolytischer Trennungsmechanismus nach Anspruch 1, wobei das Absorptionselement (102) aus einem Hydrogelmaterial hergestellt ist.

3. Elektrolytischer Trennungsmechanismus nach Anspruch 2, wobei das Absorptionselement (102) das Trennungselement umgibt.

4. Elektrolytischer Trennungsmechanismus nach Anspruch 3, wobei das Absorptionselement (102) eine spiralförmige Struktur oder ein hohles Rohr ist, das über das Trennungselement (103) geschoben ist.

5. Elektrolytischer Trennungsmechanismus nach Anspruch 2, wobei das Absorptionselement (102) über das Trennungselement geschichtet ist.

6. Elektrolytischer Trennungsmechanismus nach Anspruch 2, wobei das Hydrogelmaterial ausgewählt ist aus einem der Folgenden oder aus einer Kombination von mehr als einem der Folgenden: Hydrogel auf Basis von Cellulose und Derivaten davon; Gelatine-modifizierte Hydrogele; vernetzte Hydrogele auf Basis von Chitosan und Derivaten davon; vernetzte Hydrogele auf Basis von Hyaluronsäure und modifizierten Formen davon; vernetzte Hydrogele auf Basis von Polyethylenglycol und Derivaten davon; vernetzte Hydrogele auf Basis von Poly(vinylalkohol) und Derivaten davon; vernetzte Hydrogele auf Basis von Poly(N-methylpyrrolidon) und Derivaten davon; polyesterbasierte Hydrogele; vernetztes Hydrogel auf Basis von Polyacrylamid und Derivaten davon; vernetzte quellbare Polymere, die von einem oder mehreren polymerisierbaren ungesättigten Carbonsäuremonomeren abgeleitet sind, die olefinische Bindungen enthalten; und Hydrogel auf Basis von Hydroxyethylmethacrylat und Derivaten davon.

7. Elektrolytische Trennungsvorrichtung, umfassend den elektrolytischen Trennungsmechanismus nach einem der Ansprüche 1 bis 6, wobei die elektrolytische Trennungsvorrichtung ferner umfasst:
einen Katheter (301); und
eine Schubstange (201), die mit einem proximalen Ende des elektrolytischen Trennungsmechanismus gekoppelt ist,
wobei sowohl der elektrolytische Trennungsmechanismus als auch die Schubstange (201) beweglich in dem Katheter (301) aufgenommen sind, und wobei die Schubstange (201) ausgebildet ist, mit dem Katheter (301) zusammenzuwirken, um den elektrolytischen Trennungsmechanismus zu einem Wirkort zu befördern.

8. Elektrolytische Trennungsvorrichtung nach Anspruch 7, wobei die Schubstange (201) an einem distalen Ende derselben mit einem flexiblen Element (202) versehen ist, und wobei die Schubstange (201) mit dem elektrolytischen Trennungsmechanismus durch das flexible Element (202) gekoppelt ist.

9. Elektrolytische Trennungsvorrichtung nach Anspruch 8, wobei sowohl die Schubstange (201) als auch das flexible Element (202) eine hohle Struktur sind, wobei das elektrisch Leitungselement (101) in der Schubstange (201) und in dem flexiblen Element (202) aufgenommen ist, wobei das kathodisch leitfähige Element von einem zweiten isolierenden Element bedeckt ist, um die Schubstange (201) von dem flexiblen Element (202) zu isolieren, und wobei das kathodisch leitfähige Element ein distales Ende aufweist, das von dem zweiten isolierenden Element freiliegt und das mit dem Trennungselement (103) durch das Absorptionselement (102) elektrisch verbindbar ist.

10. Elektrolytische Trennungsvorrichtung nach Anspruch 8, wobei sowohl die Schubstange (201) als auch das flexible Element (202) eine hohle Struktur sind, wobei das anodisch leitfähige Element in der Schubstange (201) und in dem flexiblen Element (202) aufgenommen ist, wobei die Schubstange (201) und das flexible Element (202) zusammen als das kathodisch leitende Element ausgebildet sind.

11. Elektrolytische Trennungsvorrichtung nach Anspruch 9 oder 10, wobei das Absorptionselement (102) und das flexible Element (202) Seite an Seite entlang einer axialen Richtung angeordnet sind, und wobei das Absorptionselement (102) an einem distalen Ende des flexiblen Elements (202) angeordnet ist.

12. Elektrolytische Trennungsvorrichtung nach Anspruch 9 oder 10, wobei das Absorptionselement (102) einen proximalen Endabschnitt aufweist, der in dem flexiblen Element (202) aufgenommen ist, und wobei das Absorptionselement (102) ein distales Ende aufweist, das aus einem distalen Ende des flexiblen Elements (202) herausragt.

13. Elektrolytische Trennungsvorrichtung nach Anspruch 9 oder 10, wobei das Absorptionselement (102) vollständig in dem flexiblen Element (202) aufgenommen ist.

14. Elektrolytische Trennungsvorrichtung nach Anspruch 8, wobei das flexible Element (202) einen ersten strahlenundurchlässigen Abschnitt (203) aufweist und ein distales Ende des Katheters (303) einen zweiten strahlenundurchlässigen Abschnitt (302) aufweist, und wobei jeder des ersten und zweiten strahlenundurchlässigen Abschnitts (203, 302) aus einem strahlenundurchlässigen Material hergestellt ist.

## Revendications

1. Mécanisme de détachement électrolytique destiné à coopérer avec un appareil de détachement électrolytique pour obtenir un détachement électrolytique d'un implant, comprenant :
l'implant ;
un élément de détachement (103) ayant une extrémité distale accouplée à l'implant, dans lequel l'élément de détachement (103) est configuré pour être traversé par un courant et dissous électrolytiquement, éliminant ainsi l'accouplement entre l'implant et l'élément de détachement (103) ;
un élément de conduction électrique (101), comprenant :
un élément conducteur anodique recouvert par un premier élément isolant, dans lequel l'élément conducteur anodique a une extrémité distale accouplée à une extrémité proximale de l'élément de détachement (103) et une extrémité proximale configurée pour être accouplée à une borne positive de l'appareil de détachement électrolytique ; et
un élément conducteur cathodique ayant une extrémité proximale configurée pour être accouplée à une borne négative de l'appareil de détachement électrolytique, dans lequel l'élément conducteur cathodique est électriquement isolé de l'élément conducteur anodique par le premier élément isolant ;
**caractérisé en ce que** le mécanisme de détachement électrolytique comprend en outre
un élément d'absorption (102) configuré pour, lorsqu'il absorbe des électrolytes, fournir une conduction électrique entre l'élément de détachement (103) et l'élément conducteur cathodique.

2. Mécanisme de détachement électrolytique selon la revendication 1, dans lequel l'élément d'absorption (102) est réalisé en matériau hydrogel.

3. Mécanisme de détachement électrolytique selon la revendication 2, dans lequel l'élément d'absorption (102) entoure l'élément de détachement.

4. Mécanisme de détachement électrolytique selon la revendication 3, dans lequel l'élément d'absorption (102) est une structure en spirale ou un tube creux manchonné(e) sur l'élément de détachement (103).

5. Mécanisme de détachement électrolytique selon la revendication 2, dans lequel l'élément d'absorption (102) est revêtu sur l'élément de détachement.

6. Mécanisme de détachement électrolytique selon la revendication 2, dans lequel le matériau d'hydrogel est un élément ou une combinaison de plusieurs éléments choisis parmi : un hydrogel à base de cellulose et des dérivés de celui-ci ; des hydrogels modifiés par la gélatine ; des hydrogels réticulés à base de chitosane et des dérivés de ceux-ci ; des hydrogels réticulés à base d'acide hyaluronique et des formes modifiées de ceux-ci ; des hydrogels réticulés à base de polyéthylène glycol et des dérivés de ceux-ci ; des hydrogels réticulés à base d'alcool polyvinylique et des dérivés de ceux-ci ; des hydrogels réticulés à base de poly(N-méthylpyrrolidone) et des dérivés de ceux-ci ; des hydrogels à base de polyester ; un hydrogel réticulé à base de polyacrylamide et des dérivés de celui-ci ; des polymères gonflables réticulés dérivés d'un ou plusieurs monomères d'acide carboxylique insaturés polymérisables contenant des liaisons oléfiniques ; et un hydrogel à base de méthacrylate d'hydroxyéthyle et des dérivés de celui-ci.

7. Dispositif de détachement électrolytique comprenant le mécanisme de détachement électrolytique selon l'une quelconque des revendications 1 à 6, dans lequel le dispositif de détachement électrolytique comprend en outre :
un cathéter (301) ; et
une tige de poussée (201) accouplée à une extrémité proximale du mécanisme de détachement électrolytique,
dans lequel chacun du mécanisme de détachement électrolytique et de la tige de poussée (201) est reçu de manière mobile dans le cathéter (301), et dans lequel la tige de poussée (201) est configurée pour coopérer avec le cathéter (301) pour poser le mécanisme de détachement électrolytique sur un site cible.

8. Dispositif de détachement électrolytique selon la revendication 7, dans lequel la tige de poussée (201) est pourvue à son extrémité distale d'un élément flexible (202), et dans lequel la tige de poussée (201) est accouplée au mécanisme de détachement électrolytique par l'élément flexible (202).

9. Dispositif de détachement électrolytique selon la revendication 8, dans lequel chacun de la tige de poussée (201) et de l'élément flexible (202) est une structure creuse, dans lequel l'élément de conduction électrique (101) est reçu dans la tige de poussée (201) et dans l'élément flexible (202), dans lequel l'élément conducteur cathodique est recouvert par un second élément isolant pour isoler la tige de poussée (201) de l'élément flexible (202), et dans lequel l'élément conducteur cathodique a une extrémité distale apparente depuis le second élément isolant et pouvant être connectée électriquement à l'élément de détachement (103) par l'élément d'absorption (102).

10. Dispositif de détachement électrolytique selon la revendication 8, dans lequel chacun de la tige de poussée (201) et de l'élément flexible (202) est une structure creuse, dans lequel l'élément conducteur anodique est reçu dans la tige de poussée (201) et dans l'élément flexible (202), la tige de poussée (201) et l'élément flexible (202) étant configurés ensemble en tant qu'élément conducteur cathodique.

11. Dispositif de détachement électrolytique selon la revendication 9 ou 10, dans lequel l'élément d'absorption (102) et l'élément flexible (202) sont agencés côte à côte le long d'une direction axiale, et dans lequel l'élément d'absorption (102) est situé sur une extrémité distale de l'élément flexible (202).

12. Dispositif de détachement électrolytique selon la revendication 9 ou 10, dans lequel l'élément d'absorption (102) a une partie extrémité proximale reçue dans l'élément flexible (202), et dans lequel l'élément d'absorption (102) a une extrémité distale faisant saillie hors d'une extrémité distale de l'élément flexible (202).

13. Dispositif de détachement électrolytique selon la revendication 9 ou 10, dans lequel l'élément d'absorption (102) est entièrement reçu dans l'élément flexible (202).

14. Dispositif de détachement électrolytique selon la revendication 8, dans lequel l'élément flexible (202) a une première section radio-opaque (203) et une extrémité distale du cathéter (303) a une seconde section radio-opaque (302), et dans lequel chacune des première et seconde sections radio-opaques (203, 302) est réalisée en matériau radio-opaque.
